# EUROPÄISCHE PATENTANMELDUNG

(11) **EP 2 248 463 A1**
(43) Veröffentlichungstag der Anmeldung: **10.11.2010**
(21) Anmeldenummer: 09159834.2
(22) Anmeldetag: 09.05.2009
(51) Int. Cl.: A61B 5/00, A61B 5/151

(54) **Testeinheit zum Einsatz in einem Testgerät und Testsystem**

(71) Anmelder: F. Hoffmann-La Roche AG, 4070 Basel (CH); Roche Diagnostics GmbH, 68305 Mannheim (DE)
(72) Erfinder: List, Hans, 64754 Hesseneck-Kailbach (DE)
(74) Vertreter: Pfiz, Thomas

(57) **Zusammenfassung**

Die Erfindung betrifft ein Testsystem mit einem Testgerät (12), das einen Stechantrieb (14) und eine optische Messeinrichtung (16) aufweist, und mindestens einem als Einwegartikel vorzugsweise in einem Magazin (18) in das Testgerät (12) eingesetzten Testeinheit (20). Erfindungsgemäß wird vorgeschlagen, dass die Messeinrichtung (16) über einen geräteseitigen Optikadapter (68) unmittelbar an ein Nachweiselement (24) der Testeinheit (20) ankoppelbar ist, wobei ein freies Ende des federgelagerten Optikadapters (68) kraftschlüssig gegen das Nachweiselement (24) anliegt.

## Beschreibung

### Beschreibung

Die Erfindung betrifft eine Testeinheit zum Einsatz in einem Testgerät für eine Einmaluntersuchung einer Körperflüssigkeit bestehend aus einem in die Haut eines Benutzers einstechbaren Stechelement und einem mit Körperflüssigkeit aus der Haut beaufschlagbaren analytischen Nachweiselement, das aus einer mit einem Analyten in der Körperflüssigkeit reagierenden Reagenzschicht und einem mit der Reagenzschicht beschichteten transparenten Trägerplättchen besteht. Die Erfindung betrifft weiter ein Testsystem zur entsprechenden Verarbeitung derartiger Testeinheiten.

Aus der WO 2007/045412 der Anmelder ist ein solches Testsystem bekannt, bei welchem die disposiblen Testeinheiten mit verschiebefest integrierten Lichtleitern versehen sind, um das Messlicht aus einer Messzone zu einem Schaftende eines Stechteils zu leiten, an welchem zusätzlich ein Kopplungsteil zur mechanischen Kopplung angespritzt ist. In einer Ausgestaltung wird dort vorgeschlagen, dass die optische Ankopplung an die Rückseite des Testfelds über eine Trägerfolie erfolgen kann, die fest mit den distalen Enden der integrierten Lichtleiter verbunden ist. Eine solche Anordnung ermöglicht es, einen aufwändigen Probentransport zu vermeiden, indem die Messung direkt in der Sammelzone durchgeführt wird, bedingt aber zugleich eine erhöhte Komplexität der nur für eine Einmalmessung einsetzbaren Testeinheiten.

Ausgehend hiervon liegt der Erfindung die Aufgabe zugrunde, die im Stand der Technik bekannten Einheiten und Systeme weiter zu verbessern und insbesondere auch für die Massenherstellung miniaturisierter Einwegteile eine günstige Bauform bei gleichzeitig zuverlässiger Messwerterfassung anzugeben.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die Testeinheit frei von Lichtwellenleitern zu halten und stattdessen eine im Gerät bereitgestellte Lichtleitstruktur kraftschlüssig anzudocken. Dementsprechend wird erfindungsgemäß vorgeschlagen, dass das Trägerplättchen an einer von der Reagenzschicht abgewandten Anschlussfläche eine mit einem geräteseitigen Optikadapter durch Andrücken direkt in Anlage bringbare optische Schnittstelle zur photometrischen Messwerterfassung bildet. Somit wird auf eine zusätzliche Lichtleiterstruktur in dem Einwegteil verzichtet, wodurch neben dem verringerten Herstellungsaufwand besondere Vorteile bezüglich der Baugröße erreicht werden, die auch für eine Magazinierung einer großen Anzahl von Verbrauchseinheiten bedeutsam ist. Dabei ist auch zu bedenken, dass für eine sichere Messwerterfassung ggf. mehrere parallele Lichtleiter erforderlich sind, welche die Dimensionierung deutlich erhöhen würden. Zudem ist keine Kopplung zwischen geräteseitigen und verbrauchsteilseitigen Lichtleitern erforderlich, so dass eine zusätzliche verlustbehaftete Schnittstelle eingespart wird.

Um ein optimales Messsignal zu bekommen, wurde als besonders bedeutsam erkannt, dass die Dicke des Trägerplättchens auf die optischen Eigenschaften der Lichtleiter im Optikadapter, insbesondere deren Durchmesser, numerische Apertur und gegenseitiger Abstand abgestimmt sein sollte. Zu berücksichtigen ist auch der Brechungsindex des Trägerplättchens. Außerdem sollte es möglich sein, handelsübliche Folien als Ausgangsmaterial einzusetzen. Unter Beachtung dieser Randbedingungen ist es von besonderem Vorteil, wenn das Trägerplättchen eine Dicke von weniger als 500 Mikrometer, vorzugsweise etwa 200 bis 300 Mikrometer besitzt.

Für die gezielte Verarbeitung kleinster Probenmengen unter Berücksichtigung einer kapillaraktiven Bereitstellung ist es weiterhin vorteilhaft, wenn das Trägerplättchen als Zuschnitt aus einer Folie gebildet ist und eine Breitseitenfläche von weniger als 5 mm², bevorzugt weniger als 1 mm² aufweist.

Beide Aspekte der opto-mechanischen Kopplung können dadurch besonders vorteilhaft berücksichtigt werden, dass das Stechelement ein Widerlager zur Abstützung einer kraftschlüssigen Verbindung zwischen der optischen Schnittstelle und dem Optikadapter aufweist.

Für eine schnelle und genaue Stechbewegung ist es günstig, wenn das Stechelement eine mit einem geräteseitigen Stechantrieb in Eingriff bringbare Kopplungsstruktur aufweist. Eine weitere Verbesserung in dieser Hinsicht ergibt sich dadurch, dass das Stechelement zwei quer zu einer Stechachse gegensinnig auslenkbare, elastische Kopplungsarme aufweist, wobei die Kopplungsarme bei der Stechbewegung des Stechelements aus einer vorgespannten Freigabestellung in eine entspannte Kopplungsstellung bringbar sind.

Herstellungstechnisch ist besonders vorteilhaft, wenn das Stechelement als Flachformteil einstückig aus einem Blechmaterial gebildet, insbesondere geätzt ist, so dass keine zusätzlichen Funktionsteile angeformt werden müssen.

Eine auch für die Gerätekopplung besonders günstige Bauform wird dadurch geschaffen, dass das Stechelement ein U-förmiges Basisteil und ein an dem Basisteil angeformtes, vorzugsweise mit einer Kapillarrinne versehenes nadelförmiges Stechorgan aufweist.

Ein weiterer Erfindungsaspekt besteht in einem Testsystem umfassend ein Testgerät und mindestens einer darin als Einwegartikel eingesetzten Testeinheit, wobei die Messeinrichtung über einen geräteseitigen Optikadapter unmittelbar an das Nachweiselement der Testeinheit ankoppelbar ist und ein freies Ende des federgelagerten Optikadapters kraftschlüssig gegen das Nachweiselement anliegt. Durch den Kraftschluss können Herstellungstoleranzen ausgeglichen werden und zugleich auch während der Stechbewegung zuverlässig Messsignale abgegriffen werden.

Eine vorteilhafte Ausführung sieht hierbei vor, dass der Optikadapter über ein vorzugsweise als Feder ausgebildetes Rückstellmittel abgestützt ist und unter der Wirkung einer von dem Rückstellmittel ausgeübten Kraft gegen das Nachweiselement andrückbar ist.

Eine weitere Verbesserung wird dadurch erreicht, dass der Optikadapter in einem Antriebsstößel des Stechantriebs verschieblich gelagert ist, und dass der Antriebsstößel vorzugsweise über Greifer zugfest mit der Testeinheit koppelbar ist.

Um zunächst eine definierte mechanische Ankopplung zu ermöglichen, ist es von Vorteil, wenn der Optikadapter im Zuge einer durch den Stechantrieb erzeugten Vorschubbewegung durch ein wegabhängig geschaltetes Steuermittel mit einer in Vorschubrichtung wirkenden Federkraft zur optischen Ankopplung an das Nachweiselement beaufschlagbar ist.

Für einen direkten Signalabgriff an dem analytischen Bauteil ist es vorteilhaft, wenn der Optikadapter einen oder mehrere nebeneinander verlaufende Lichtleiter aufweist, und wenn die Lichtleiter stirnseitig auf Stoß mit der Anschlussfläche des Trägerplättchens verbindbar sind.

Für eine sichere Messwerterfassung ist es vorteilhaft, wenn der Optikadapter an einem von der Testeinheit abgewandten Anschlussende mit einer opto-elektronischen Baugruppe der Messeinrichtung verschiebefest verbunden ist.

Vorteilhafterweise ist die Testeinheit in einer Führung vorzugsweise in einer Magazinkammer gelagert, wobei die Führung eine insbesondere bogenförmige Führungsbahn zur Herstellung eines Formschlusses zwischen der Testeinheit und dem Stechantrieb im Zuge einer Stechbewegung aufweist, so dass eine selbsttätige mechanische Ankopplung erreicht wird.

Im Folgenden wird die Erfindung anhand der in der Zeichnung schematisch dargestellten Ausführungsbeispiele näher erläutert. Es zeigen
- Fig. 1: ein Blutzucker-Testsystem mit einem darin ein- gesetzten, teilweise aufgebrochen gezeigten Ma- gazin für Testeinheiten in einer perspektivi- schen Ansicht;
- Fig. 2: das Testsystem nach Fig. 1 mit aktivierter Testeinheit in von dem Magazin freigestellter Darstellung;
- Fig. 3: eine an einen Stechantrieb mechanisch angekop- pelte Testeinheit gemäß Fig. 2;
- Fig. 4 und 5: eine ausschnittsweise Seitenansicht und Unterseitenansicht der Anordnung nach Fig. 3;
- Fig. 6 bis 8: die zusätzlich optisch angekoppelte Testeinheit in vorgeschobener Stechstellung entsprechend den Darstellungen gemäß Fig. 3 bis 5;
- Fig. 9: eine isolierte Testeinheit in perspektivischer Ansicht;
- Fig. 10: das Magazin nach Fig. 1 in ausschnittsweiser Draufsicht; und
- Fig. 11: den Strahlengang bei einem an die Testeinheit angekoppelten Optikadapter in schaubildlicher Darstellung.

Das in der Zeichnung dargestellte Testsystem 10 zur Blutzuckermessung umfasst ein kompaktes tragbares Testgerät 12 mit eingebautem Stechantrieb 14 und Messeinrichtung 16 sowie ein auswechselbares scheibenförmiges Magazin 18 mit einer Vielzahl von darin befindlichen Testeinheiten 20 als Verbrauchsartikel zur Durchführung jeweils eines Blutzuckertests, wobei die Testeinheiten 20 in einem besonders einfachen Aufbau aus einem Stechelement 22 mit integriertem Nachweiselement 24 für eine Probengewinnung und unmittelbare photometrische Messwerterfassung bestehen.

Dadurch wird die Selbstbestimmung der Blutzuckerkonzentration in einem vollautomatischen Messablauf auch für Laien mit hohem Handhabungskomfort zuverlässig durchführbar.

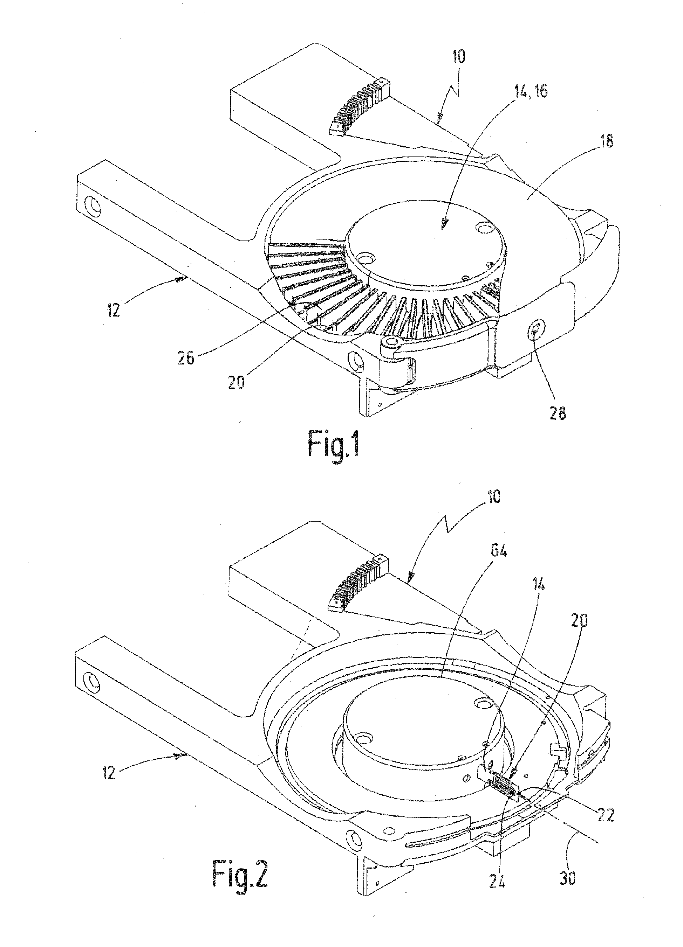

Wie in Fig. 1 veranschaulicht, sind eine Vielzahl von Testeinheiten 20 in jeweiligen Magazinkammern 26 durch Drehung des Magazins 18 sukzessive in eine aktive Gebrauchsposition bezüglich einer mit einer Durchstechöffnung versehenen Auflage 28 zur Fingerpositionierung eines Benutzers bringbar. Der in die aktiv positionierte Magazinkammer 26 eingreifende Stechantrieb 14 ermöglicht dabei eine hin- und hergehende Bewegung der Testeinheit 20 längs einer Stechachse 30, wie es in Fig. 2 veranschaulicht ist. Nach erfolgter Blutaufnahme und Messwerterfassung kann die gebrauchte Testeinheit 20 wieder in die Magazinkammer 26 zurückgezogen und dadurch entsorgt werden.

Generell können solche Messungen außer an der Fingerbeere auch an anderen Körperteilen, beispielsweise im weniger schmerzempfindlichen Arm- oder Bauchbereich vorgenommen werden, wobei als Körperflüssigkeit für die Probennahme aus der Haut neben Kapillarblut auch Gewebeflüssigkeit oder Mischungen davon in Frage kommen.

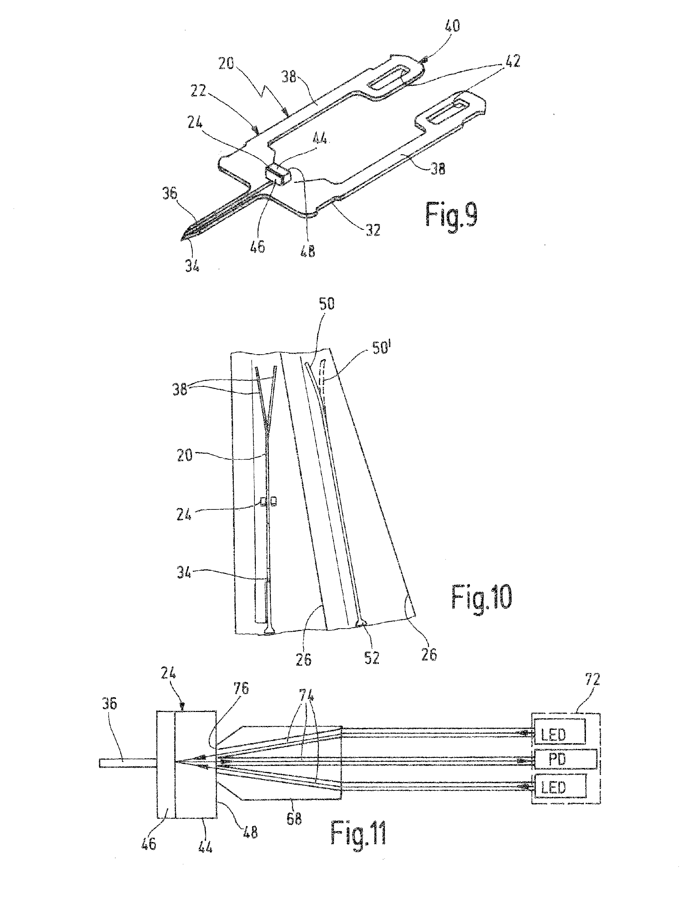

Fig. 9 zeigt eine einzelne Testeinheit 20 bestehend aus Stechelement 22 und Nachweiselement 24. Das Stechelement 22 ist als Flachformteil einstückig aus einem Edelstahlblech geätzt und weist ein U-förmiges Basisteil 32 und ein mittig distal daran angeformtes nadelförmiges Stechorgan 34 auf, das mit einem rinnenförmigen Kapillarkanal 36 zur Blutaufnahme beim Hauteinstich versehen ist. Die proximal abstehenden U-Schenkel bilden Kopplungsarme 38, die quer zur Stechachse 30 gegensinnig auslenkbar sind, wie es nachstehend näher erläutert wird. An den freien Enden der Kopplungsarme 38 ist eine Kopplungsstruktur 40 zum Einhaken des Stechantriebs 14 ausgebildet, wobei die Ösen 42 ein Widerlager zur Abstützung gegen eine bei der optischen Ankopplung in distaler Richtung eingeleitete Federdruckkraft bilden. Wie aus Fig. 9 weiter zu ersehen ist, besteht das Nachweiselement 24 aus einem Trägerplättchen 44, das in das Stechelement 22 fest eingesetzt ist und an seiner dem Kanal 36 zugewandten Seite mit einer Reagenzschicht 46 versehen ist. Diese reagiert als an sich bekanntes enzymatisches System auf einen Analyten (Glucose) in der anströmenden Blutflüssigkeit irreversibel durch eine Farbänderung, die rückseitig durch das transparente Trägerplättchen 44 hindurch reflektometrisch erfassbar ist. Das Trägerplättchen 44 bildet somit mit seiner von der Reagenzschicht 46 abgewandten rückseitigen Anschlussfläche 48 eine optische Schnittstelle zur direkten geräteseitigen Ankopplung, ohne dass zusätzliche Lichtleiter oder optische Bauteile in dem Verbrauchsteil notwendig wären. Zweckmäßig besteht das Trägerplättchen aus einem Folienzuschnitt beispielsweise aus PET, PC oder PMMA.

Fig.10 zeigt ausschnittsweise zwei Magazinkammern 26, wobei nur die linke Kammer mit einer Testeinheit 20 bestückt ist. Deren Kopplungsarme 38 sind zur mechanischen Ankopplung an den Stechantrieb 14 in einer vorgespannten Ausgangsstellung gehalten. Zu diesem Zweck weist das Magazin 18 deckel- und bodenseitige Führungsbahnen 50, 50' auf, die in einem proximalen Endabschnitt bogenförmig auseinanderlaufen und somit für eine entsprechende Aufspreizung der Kopplungsarme 38 sorgen. Beim radialen Vorschub in Richtung des distalen Führungsbahnendes 52 schwenken die Kopplungsarme 38 in eine gemeinsame Führungsebene ein, in der die Ösen 42 in den Stechantrieb 14 selbsttätig einhaken, während die Vorspannung aufgehoben ist, so dass ein weitgehend reibungsfreier Stechvorgang möglich ist.

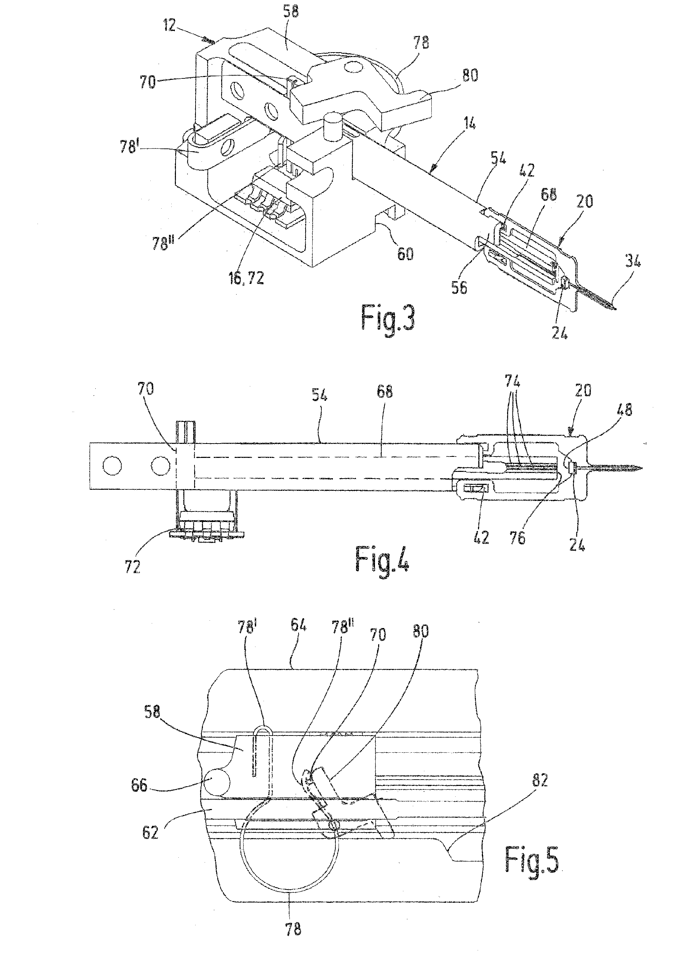

Die Figuren 3 bis 5 zeigen diese Kopplungsstellung der Testeinheit 20 in einer Anfangsphase, in der noch keine optische Verbindung zu dem Nachweiselement 24 besteht. Hierbei ist ein Antriebstößel 54 des Stechantriebs 14 mit seinem Greiferende 56 in die Ösen 42 der Testeinheit 20 mit Längsspiel eingehakt, wobei das Stechorgan 34 sich noch in der Magazinkammer 26 befindet. Der Antriebstößel 54 ist an einem Schlitten 58 befestigt, der an einer nach unten offenen Führungsnut 60 auf eine gerätefeste Schiene 62 in dem Gehäusedom 64 aufsetzbar ist und darauf mittels eines Antriebszapfens 66 hin und her verschiebbar ist, um die Stechbewegung zu übertragen. Der Antriebszapfen 66 ist hierbei in einer nicht gezeigten Kulissenscheibe geführt, die unter motorischer Drehung ein gewünschtes Stechprofil vermittelt.

Wie insbesondere aus Fig. 2 zu ersehen ist, ermöglicht ein geräteseitiger Optikadapter 68 eine unmittelbar optische Ankopplung der Messeinrichtung 16 an das Nachweiselement 24 der Testeinheit 20. Der Optikadapter 68 ist in Form eines Innenstößels in dem Antriebstößel 54 begrenzt verschieblich gelagert. An seinem von der Testeinheit 20 abgewandten, T-förmigen Anschlussende 70 ist der Optikadapter 68 mit einer opto-elektronischen Baugruppe 72 starr verbunden. Diese Baugruppe 72 enthält als Teil der Messeinrichtung 16 einen Lichtsender und einen Lichtdetektor, so dass ein robustes elektrisches Ausgangssignal zur weiteren Verarbeitung bereitgestellt werden kann. Zur direkten optischen Ankopplung an das Nachweiselement 24 verlaufen in dem Optikadapter 68 mehrere Lichtleiter 74 nebeneinander. Diese enden an einer freien distalen Stirnfläche 76, die auf Stoß mit der zunächst beabstandeten Anschlussfläche 48 des Nachweiselements 24 verbindbar ist.

Um die optische Verbindung zuverlässig durch einen Kraftschluss herstellen zu können, ist eine schwanenhalsförmige Blattfeder 78 als Rückstellmittel vorgesehen, die an ihrem einen Federende 78' an dem Schlitten 58 befestigt ist und an ihrem anderen Federende 78" gegen den unteren Schenkel des T-förmigen Anschlussendes 70 des Optikadapters 68 andrückt (Fig. 3).

Die Vorspannung der Feder 78 wird erst im Zuge der Vorschubbewegung nach erfolgtem Einhaken des Greiferendes 56 auf den Optikadapter 68 aufgeschaltet. Zu diesem Zweck ist ein Stellhebel 80 als Steuermittel an dem Schlitten 58 schwenkbar gelagert. Wie aus Fig. 5 ersichtlich, tastet der Stellhebel 80 beim Stechvorschub eine abgestufte Steuerbahn 82 ab, die neben der Schiene 62 verläuft. In dem Anfangsbereich der Steuerbahn 82 wird das Anschlussende 70 über den Stellhebel 80 abgestützt, so dass die Feder 78 keinen Vorschub des Optikadapters 68 bewirkt.

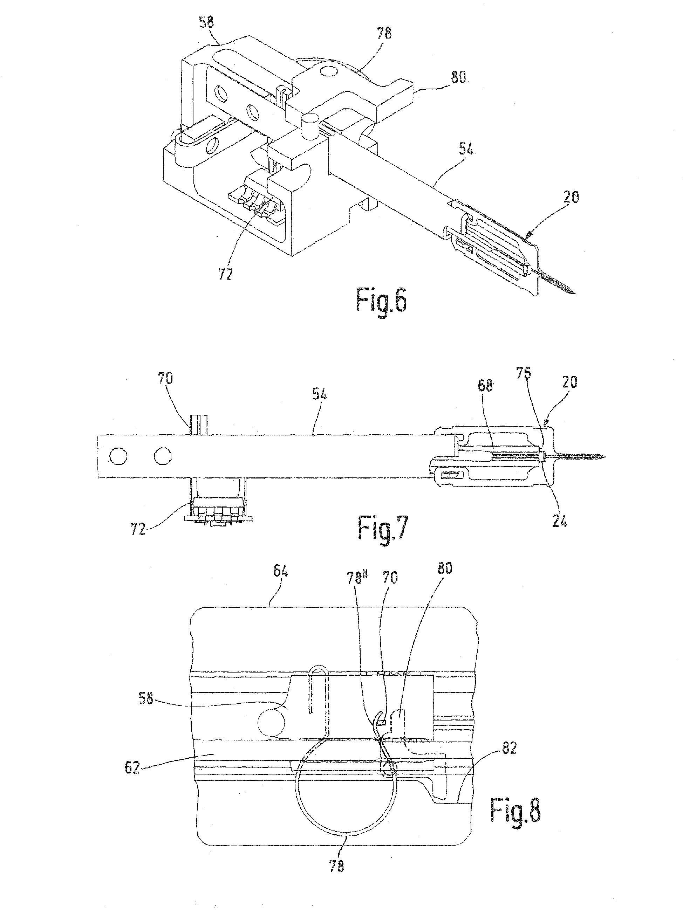

Wie in Fig. 6 bis 8 veranschaulicht, gelangt der Optikadapter 68 mit dem Nachweiselement 24 in kraftschlüssige Verbindung, sobald der Steuerhebel 80 im Zuge des Vorschubs die Stufe der Steuerbahn 80 abgetastet hat. Die Stirnfläche 76 wird dann unter der Vorspannkraft der Feder 78 rückseitig gegen das Nachweiselement angedrückt, um eine direkte Ein- bzw. Auskopplung des Messlichts zu ermöglichen. Hierbei wird das Greiferende 56 in eine zugfeste Verbindung mit den Langlöchern bzw. Ösen 42 gebracht, welche die Reaktionskraft des Federvorschubs aufnehmen und somit als Widerlager wirken. Auf diese Weise kann im verspannten Zustand die Stechbewegung vollzogen werden, wobei in dem Hauteinstich eine mikroskopische Blutmenge aufgenommen und einmalig untersucht wird.

Fig. 11 zeigt den Strahlengang bei der Messung schematisch vereinfacht. Das Licht von zwei Leuchtdioden LED mit ggf. unterschiedlicher Wellenlänge wird über zugeordnete Lichtleiter 74 zentral hinter der Kanal 36 eingestrahlt. Zur genaueren punktförmigen Ausrichtung können die Lichtleiter 74 zu dem freien Ende 76 hin so zusammenlaufen, dass der gegenseitige Abstand kleiner ist als auf der Einstrahlseite. Das an der Reagenzschicht 46 gestreute Messlicht wird ebenfalls über einen zugeordneten mittigen Lichtleiter 74 auf eine Photodiode PD als Detektor zurückgeleitet. Um ein möglichst großes Nutzsignal zu erhalten und dabei eine geringe Abhängigkeit von Abstandstoleranzen zu gewährleisten, ist die Dicke der Trägerfolie 44 auf etwa 200 bis 300 Mikrometer begrenzt. Zugleich kann die Anschlussfläche auf etwa 0,6 x 0,6 mm² eingegrenzt werden, so dass auch kleinste Füllmengen in der Kapillarrinne 36 zu einer hinrechenden Benetzung der Reagenzschicht 46 führen.

Durch das beschriebene System 10 kann ein komplexer Messablauf mit reduzierten mechanischen und optischen Komponenten auf der Seite des als Einheit verarbeiteten Verbrauchsteils 20 verwirklicht werden. Der Benutzer muss nur einen Handhabungsschritt für eine Probengewinnung und Messung durchführen und sich um die Entsorgung der Verbrauchsteile keine Gedanken machen. Durch die federnde Ankopplung des Optikadapters 68 in Richtung der Stechachse 30 können Toleranzen ausgeglichen werden, ohne dass Signalverluste auftreten.

## Patentansprüche

1. Testeinheit zum Einsatz in einem Testgerät (12) für eine Einmaluntersuchung einer Körperflüssigkeit mit einem in die Haut eines Benutzers einstechbaren Stechelement (22) und einem mit Körperflüssigkeit aus der Haut beaufschlagbaren analytischen Nachweiselement (24), das aus einer mit einem Analyten in der Körperflüssigkeit reagierenden Reagenzschicht (46) und einem mit der Reagenzschicht (46) beschichteten transparenten Trägerplättchen (44) besteht, **dadurch gekennzeichnet, dass** das Trägerplättchen (44) an einer von der Reagenzschicht (46) abgewandten Anschlussfläche (48) eine mit einem geräteseitigen Optikadapter (68) in Anlage bringbare optische Schnittstelle zur photometrischen Messwerterfassung bildet.

2. Testeinheit nach Anspruch 1, **dadurch gekennzeichnet, dass** das Trägerplättchen (44) eine Dicke von weniger als 500 Mikrometer, vorzugsweise von 200 bis 300 Mikrometer besitzt.

3. Testeinheit nach Anspruch 1 oder 2, **dadurch gekennzeichnet, dass** das Trägerplättchen (44) als Zuschnitt aus einer Folie gebildet ist und eine Anschlussfläche (48) von weniger als 5 mm², bevorzugt weniger als 1 mm² aufweist.

4. Testeinheit nach einem der Ansprüche 1 bis 3, **dadurch gekennzeichnet, dass** das Stechelement (22) ein Widerlager (42) zur Abstützung einer kraftschlüssigen Verbindung zwischen der optischen Schnittstelle und dem Optikadapter (68) aufweist.

5. Testeinheit nach einem der Ansprüche 1 bis 4, **dadurch gekennzeichnet, dass** das Stechelement (22) eine mit einem geräteseitigen Stechantrieb (14) in Eingriff bringbare Kopplungsstruktur (40) aufweist.

6. Testeinheit nach einem der Ansprüche 1 bis 5, **dadurch gekennzeichnet, dass** das Stechelement (22) zwei quer zu einer Stechachse (30) gegensinnig auslenkbare, elastische Kopplungsarme (38) aufweist, wobei die Kopplungsarme (38) bei der Stechbewegung des Stechelements (22) aus einer vorgespannten Freigabestellung in eine entspannte Kopplungsstellung bringbar sind.

7. Testeinheit nach einem der Ansprüche 1 bis 6, **dadurch gekennzeichnet, dass** das Stechelement (22) als Flachformteil einstückig aus einem Blechmaterial gebildet, insbesondere geätzt ist.

8. Testeinheit nach einem der Ansprüche 1 bis 7, **dadurch gekennzeichnet, dass** das Stechelement (22) ein U-förmiges Basisteil (32) und ein an dem Basisteil (32) angeformtes, vorzugsweise mit einer Kapillarrinne (36) versehenes nadelförmiges Stechorgan (34) aufweist.

9. Testsystem mit einem Testgerät (12), das einen Stechantrieb (14) und eine optische Messeinrichtung (16) aufweist, und mindestens einem als Einwegartikel vorzugsweise in einem Magazin (18) in das Testgerät (12) eingesetzten Testeinheit (20) nach einem der vorhergehenden Ansprüche, **dadurch gekennzeichnet, dass** die Messeinrichtung (16) über einen geräteseitigen Optikadapter (68) unmittelbar an das Nachweiselement (24) der Testeinheit (20) ankoppelbar ist, wobei ein freies Ende des Optikadapters (68) kraftschlüssig gegen das Nachweiselement (24) anliegt.

10. Testsystem nach Anspruch 9, **dadurch gekennzeichnet, dass** der Optikadapter (68) über ein vorzugsweise als Feder ausgebildetes Rückstellmittel (78) abgestützt ist und unter der Wirkung einer von dem Rückstellmittel (78) ausgeübten Kraft gegen das Nachweiselement (24) andrückbar ist.

11. Testsystem nach Anspruch 9 oder 10, **dadurch gekennzeichnet, dass** der Optikadapter (68) in einem Antriebsstößel (54) des Stechantriebs (14) gelagert ist, und dass der Antriebsstößel (54) vorzugsweise über Greifer (56) zugfest mit der Testeinheit (20) koppelbar ist.

12. Testsystem nach einem der Ansprüche 9 bis 11, **dadurch gekennzeichnet, dass** der Optikadapter (68) im Zuge einer durch den Stechantrieb (14) erzeugten Vorschubbewegung durch ein wegabhängig geschaltetes Steuermittel (80) mit einer in Vorschubrichtung wirkenden Federkraft zur optischen Ankopplung an das Nachweiselement (24) beaufschlagbar ist.

13. Testsystem nach einem der Ansprüche 9 bis 12, **dadurch gekennzeichnet, dass** der Optikadapter (68) einen oder mehrere nebeneinander verlaufende Lichtleiter (74) aufweist, und dass die Lichtleiter (74) stirnseitig auf Stoß mit der Anschlussfläche (48) des Trägerplättchens (44) verbindbar sind.

14. Testsystem nach einem der Ansprüche 9 bis 13, **dadurch gekennzeichnet, dass** der Optikadapter (68) an einem von der Testeinheit (20) abgewandten Anschlussende (70) mit einer opto-elektronischen Baugruppe (72) der Messeinrichtung (16) verschiebefest verbunden ist.

15. Testsystem nach einem der Ansprüche 9 bis 14, **dadurch gekennzeichnet, dass** die Testeinheit (20) in einer Führung vorzugsweise in einer Magazinkammer (26) gelagert ist, und dass die Führung eine insbesondere bogenförmige Führungsbahn (50,50') zur Herstellung eines Formschlusses zwischen der Testeinheit (20) und dem Stechantrieb (14) im Zuge einer Stechbewegung aufweist.
